(19) Europäisches Patentamt
European Patent Office
Office européen des brevets

(11) Veröffentlichungsnummer: **0 034 711**
**A2**

(12) # EUROPÄISCHE PATENTANMELDUNG

(21) Anmeldenummer: **81100537.0**

(22) Anmeldetag: **26.01.81**

(51) Int. Cl.³: **C 07 C 143/70**
C 07 C 139/00

(30) Priorität: **08.02.80 DE 3004694**

(43) Veröffentlichungstag der Anmeldung:
**02.09.81 Patentblatt 81/35**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Dieterich, Dieter, Dr.**
**Ludwig-Girtler-Strasse 1**
**D-5090 Leverkusen 1(DE)**

(72) Erfinder: **Blank, Ulrich, Dr.**
**Am Geusfelde 35**
**D-5068 Odenthal(DE)**

(72) Erfinder: **Wolters, Erich, Dr.**
**Streffenweg 22**
**D-5162 Niederzier(DE)**

(72) Erfinder: **Langenfeld, Norbert, Dr.**
**Hofstrasse 53**
**D-5000 Köln 80(DE)**

(54) **Verfahren zur Herstellung von aromatisch gebundenen Sulfochloridgruppen aufweisenden aromatischen Isocyanaten.**

(57) Die Erfindung betrifft ein neues Verfahren zur Herstellung von Sulfochloridgruppen aufweisenden aromatischen Isocyanaten, bei welchem man aromatische Isocyanate oder Carbamidsäurechloride nacheinander oder gleichzeitig bei 0 - 170°C mit einem Sulfonierungsmittel, vorzugsweise Schwefeltrioxid oder Chlorsulfonsäure, und mit aromatischen Trihalogenmethyl-Verbindungen, vorzugsweise Benzotrichlorid oder Benzotribromid, behandelt und bei 20 - 170°C Chlorwasserstoff aus dem Reaktionsgemisch entfernt.

EP 0 034 711 A2

0034711

BAYER AKTIENGESELLSCHAFT 5090 Leverkusen-Bayerwerk
Zentralbereich
Patente Marken und Lizenzen Wr/bc/c

# Verfahren zur Herstellung von aromatisch gebundenen Sulfochloridgruppen aufweisenden aromatischen Isocyanaten

Isocyanatoarylsulfochloride sind bekannt. Sie werden gemäß der deutschen Patentschrift 947 159 durch Phosgenierung von aromatischen Aminosulfonsäuren hergestellt. Nachteilig an diesem Verfahren ist die Notwendigkeit, hochsiedende Lösungsmittel wie Dichlorbenzol und insbesondere Nitrobenzol einzusetzen und die Schwerlöslichkeit der als Ausgangsmaterial verwendeten Aminosulfonsäuren in diesen Lösungsmitteln, selbst bei den hohen angewandten Reaktionstemperaturen. Infolgedessen ist die Umsetzung langwierig und die Ausbeute der erhaltenen Isocyanatoarylsulfochloride läßt zu wünschen übrig. Dies gilt insbesondere für die Herstellung von Diisocyanatoarylsulfochloriden, von denen ein Vertreter im Beispiel 3 der o.g. Patentschrift in einer Ausbeute von 46 % der Theorie erhalten wurde. Bedingt durch die o.g. Nachteile hat das Verfahren keine technische Bedeutung erlangt.

Es ist weiterhin bekannt, einfache Arylsulfochloride durch Sulfonierung von Aromaten, z.B. mit Chlorsulfonsäure oder Gemischen aus Chlorsulfonsäure und Sulfurylchlorid herzustellen (vgl. z.B. Houben-Weyl, 4. Auflage, Band 9, Seite 563-585). Weiterhin ist es bekannt, Sulfochloride aus freien Sulfonsäuren oder deren Salzen mit Säurechloriden herzustellen. Diese aus der Literatur bekannten Verfahren sind bisher nicht oder nur mit negativem Ergebnis für die Herstellung von Isocyanatosulfochloriden aus Isocyanaten angewandt worden. Hierfür sind eine Reihe von Gründen verantwortlich:

Einmal erfordert die Sulfochlorierung mit Chlorsulfonsäure einen Überschuß dieses Reagens und daher eine Aufarbeitung in Gegenwart von Wasser, wodurch die Isocyanatgruppen angegriffen werden, zum anderen sind Isocyanatosulfonsäuren in Form der dimeren Uretdione erst in jüngster Zeit bekannt geworden und haben sich als äußerst schwierig handhabbare und extrem reaktionsfähige Verbindungen erwiesen. Darüber hinaus ist bekannt, daß Sulfonsäuregruppen mit Isocyanaten bei erhöhter Temperatur in unübersichtlicher Weise unter $CO_2$-Abspaltung reagieren, so daß unter den üblichen Bedingungen der Sulfochlorid-Herstellung in erheblichem Umfang mit anderen Reaktionen gerechnet werden mußte, z.B. auch mit Sulfon- und Anhydrid-Bildung sowie Polymerisationsreaktionen der Isocyanat-Gruppe.

Es ist aus der US-Patentschrift 3 686 301 bekannt, 3,5-Dichlorsulfo-benzoylchlorid durch Reaktion von 180°C heißer 3,5-Disulfo-benzoesäure mit der dreifach molaren Menge Benzotrichlorid zu erhalten, wobei das Benzotrichlorid während eines Zeitraumes von 3 Stunden zugegeben wird und die Temperatur während der Zugabe langsam auf 130°C reduziert wird und das Gemisch eine weitere Stunde bei 130°C gerührt wird.

Weiterhin ist aus DRP 574 836 bekannt, die Natriumsalze aromatischer Sulfonsäuren mit Benzotrichlorid zu dem entsprechenden aromatischen Sulfonsäurechlorid umzusetzen. Diese DRP enthält weiterhin den Hinweis, daß die bekannte Umsetzung freier Carbonsäuren mit Benzotrichlorid in Gegenwart von Zinkchlorid zur Herstellung der Carbonsäurechloride auf freie organische Sulfonsäuren nicht übertragbar ist.

Isocyanatoarylsulfonsäure-uretdione sind bisher nicht mit Trihalogenmethylaromaten umgesetzt worden. Die oben angeführten Bedenken gelten auch für diese Umsetzung, zumal sie bei über 100°C durchgeführt wird.

Überraschenderweise wurde nunmehr gefunden, daß auch aromatisch gebundene Isocyanatgruppen oder aromatisch gebundene Carbamidsäurechloridgruppen aufweisende Verbindungen auf besonders einfache Weise in guten Ausbeuten in aromatisch gebundene Sulfochloridgruppen aufweisende aromatische Isocyanate überführt werden können, wenn man die Ausgangsmaterialien nacheinander oder gleichzeitig mit den üblichen Sulfonierungsmitteln und aromatisch gebundene Trihalogenmethyl-Gruppen aufweisenden Verbindungen zur Reaktion bringt, obwohl wegen des Vorliegens der hochreaktiven Isocyanat- bzw. Carbamidsäurechlorid-Gruppen mit unübersichtlichen Nebenreaktionen gerechnet werden mußte. Die Verwendung der genannten Trihalogenmethyl-Aromaten hat gegenüber der Verwendung von Thionylchlorid oder Phosgen als Halogenierungsmittel darüber hinaus den Vorteil, daß kein Säureamid als Katalysator verwendet wird, und das Rohprodukt im Falle niedermolekularer Verfahrensprodukte direkt ohne Vorreinigung destilliert werden kann.

Gegenstand der vorliegenden Erfindung ist ein Verfahren zur Herstellung von aromatisch gebundene Sulfochloridgruppen aufweisenden aromatischen Isocyanaten, dadurch gekennzeichnet, daß man

Isocyanate oder Carbamidsäurechloride a), die mindestens eine aromatisch gebundene Isocyanatgruppe und/oder mindestens eine aromatisch gebundene Carbamidsäurechloridgruppe aufweisen,

nacheinander oder gleichzeitig bei 0-170°C mit

einem Sulfonierungsmittel b) und

organischen Verbindungen c) mit mindestens einer aromatisch gebundenen Trihalogenmethyl-Gruppe

behandelt und bei 20-170°C Chlorwasserstoff aus dem Reaktionsgemisch entfernt.

Geeignete Ausgangsmaterialien a) für das erfindungsgemäße Verfahren sind alle beliebigen organischen Verbindungen, die mindestens eine an einem aromatischen Ring gebundene Isocyanatgruppe und/oder mindestens eine an einem aromatischen Ring gebundene Carbamidsäurechlorid-Gruppe aufweisen und die ansonsten von ihrer Sulfonierbarkeit abgesehen unter den erfindungsgemäßen Verfahrensbedingungen inert sind.

Besonders gut geeignete Ausgangsmaterialien a) sind beispielsweise Phenylisocyanat, p-Tolylisocyanat, m-Tolylisocyanat, p-Chlorphenylisocyanat, m-Chlorphenylisocyanat, p-Bromphenylisocyanat, p-Methoxyphenylisocyanat, p-Ethoxyphenylisocyanat, p-Trifluormethylphenylisocyanat, m-Trifluormethylphenylisocyanat, 2,4-Diisocyanatotoluol bzw. dessen Gemische mit bis zu 35 Gew.-%, bezogen auf Gesamtgemisch, an 2,6-Diisocyanatotoluol, 2,2'-Diisocyanatodiphenylmethan, 2,4'-Diisocyanatodiphenylmethan, 4,4'-Diisocyanatodiphenylmethan, beliebige Mischungen aus diesen Isomeren, technische Polyphenylpolymethylen-polyisocyanate, wie sie durch Anilin/Formaldehydkondensation und anschließende Phosgenierung erhalten und z.B. in den britischen Patentschriften 874 430 und 848 671 beschrieben werden.

Anstelle der oben beispielhaft genannten Di- und Polyisocyanate können auch deren Isocyanatgruppen und Allophanat-, Biuret-, Urethan-, Harnstoff-, Carbodiimid-, Isocyanurat- oder Acylharnstoffgruppen aufweisende Modifizierungsprodukte eingesetzt werden, wie sie in an sich bekannter Weise durch eine entsprechende Modifizierungsreaktion eines Teils der

Isocyanatgruppen der Di- bzw. Polyisocyanate erhältlich sind. Es ist auch möglich, bei der technischen Herstellung aromatischer Di- bzw. Polyisocyanate anfallenden Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Isocyanatgruppen aufweisenden Verbindungen zu verwenden.

Anstelle der beispielhaft genannten Isocyanatgruppen aufweisenden Ausgangsverbindungen a) können auch beispielsweise die diesen Verbindungen entsprechenden Carbamidsäurechloride mit aromatisch gebundenen Carbamidsäurechlorid-Gruppen als Komponente a) verwendet werden. Unter "entsprechenden Carbamidsäurechloriden" sind hierbei Carbamidsäurechloridgruppen aufweisende Verbindungen zu verstehen, wie sie beispielsweise durch Anlagerung von Chlorwasserstoff an die Isocyanatgruppen der beispielhaft genannten Isocyanatgruppen aufweisenden Verbindungen erhalten werden können, und wie sie beispielsweise bei der Herstellung der beispielhaft genannten Isocyanate durch Phosgenierung der ihnen zugrundeliegenden Amine als Zwischenstufe des Phosgenierprozesses anfallen.

Als Ausgangskomponente b) kommen beim erfindungsgemäßen Verfahren beliebige Sulfonierungsmittel für aromatische Verbindungen in Betracht. Besonders gut geeignete Sulfonierungsmittel b) sind Schwefeltrioxid und/oder Chlorsulfonsäure, wobei das Schwefeltrioxid in flüssiger, gelöster oder in gasförmiger, z.B. durch Stickstoff verdünnter Form oder in Form einer Additionsverbindung an organische Verbindungen eingesetzt werden kann. Derartige Additionsverbindungen sind beispielsweise die Addukte von Schwefeltrioxid an Dioxan, Tetrahydrofuran, Diethylether oder Dimethylformamid. Bevorzugt wird gasförmiges Schwefeltrioxid eingesetzt. Gegebenenfalls mitverwendete Lösungsmittel für das Schwefeltrioxid müssen unter den Reaktionsbedingungen des erfindungsgemäßen Verfahrens gegenüber Schwefeltrioxid und auch gegenüber Isocyanatgruppen inert sein. Geeignet sind beispielsweise halogenierte oder nitrierte Kohlenwasserstoffe wie z.B. Dichlorethan, Tetrachlorethan, Methylenchlorid, Chloroform, Fluortrichlormethan, Nitromethan, Nitrobenzol, Diethylether, Dioxan, Tetrahydrofuran, Schwefeldioxid, Chlorbenzol, Dichlorbenzol oder bevorzugt überschüssige Mengen der nachstehend genannten Trihalogenmethyl-Verbindungen.

Für das erfindungsgemäße Verfahren geeignete Ausgangsmaterialien c) sind alle beliebigen organischen Verbindungen, die mindestens einen Trihalogenmethyl-Substituenten aufweisen, und die insbesondere gegenüber Isocyanatgruppen und Carbamidsäurechloridgruppen inert sind.

Unter "Trihalogenmethyl-Gruppen" sind in diesem Zusammenhang in erster Linie Tribrommethyl- oder Trichlormethyl-Gruppen, besonders bevorzugt Trichlormethylgruppen, zu verstehen. Besonders gut geeignete Ausgangsmaterialien c) sind gegebenenfalls unter den erfindungsgemäßen Verfahrensbedingungen inerte Substituenten aufweisendes Benzotribromid oder Benzotrichlorid, wobei Benzotrichlorid bzw. seine, inerte Substituenten aufweisenden, Derivate besonders bevorzugt sind.

Typische Vertreter geeigneter Ausgangskomponenten c) sind beispielsweise Verbindungen der Formel

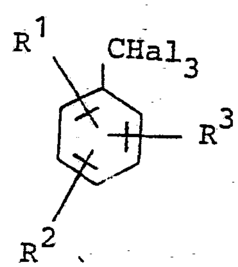

in welcher

$R^1$, $R^2$ und $R^3$ für gleiche oder verschiedene Reste stehen und Wasserstoff, $C_1$-$C_6$-Alkyl-, $C_5$-$C_7$-Cycloalkyl-, $C_6$-$C_{10}$-Aryl-, $C_7$-$C_{10}$-Aralkyl-, $C_1$-$C_4$-Alkoxy-, $C_6$-$C_{10}$-Aroxy-, $CHal_3$-, Chlor- oder Brom-Reste oder Reste der Formeln

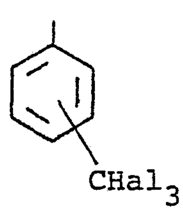 , 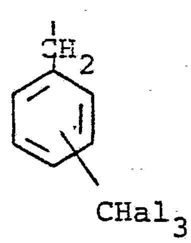 , 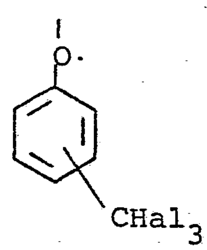 oder 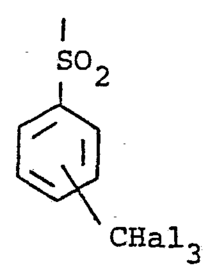

bedeuten, oder wobei zwei der Reste $R^1$ bis $R^3$, wenn sie benachbart sind, Teil eines kondensierten, cycloaliphatischen oder gegebenenfalls durch $CHal_3$-Gruppen substituierten aromatischen Rings sein können und

Hal für Chlor oder Brom steht.

Besonders bevorzugte Ausgangskomponenten c) sind solche der erstgenannten allgemeinen Formel, für welche $R^1$, $R^2$ und $R^3$ für Wasserstoff stehen, oder für welche einer der Reste für eine $C_1$-$C_2$-Alkylgruppe steht und Hal die genannte Bedeutung hat, vorzugsweise jedoch für Chlor steht.

Als typische Vertreter geeigneter Reaktionskomponenten c) seien beispielsweise folgende Verbindungen genannt:

Benzotrichlorid, 1-Chlor-2-trichlormethylbenzol, 1-Chlor-4-trichlormethylbenzol, 2,4-Dichlor-1-trichlormethylbenzol, 1,3-Bis-(Trichlormethyl)-benzol, 1,4-Bis-(Trichlormethyl)-benzol, 1-Trichlormethyl-naphthalin, 2-Trichlormethyl-naphthalin, 4-Trichlormethylbiphenyl, 4,4'-Bis-(Trichlormethyl)-biphenyl, 4-Trichlormethyl-diphenylether, 4-Trichlormethyl-diphenylmethan, Benzotribromid, 1-Brom2-tribrommethylbenzol und 1-Brom-4-tribrommethylbenzol.

Das erfindungsgemäße Verfahren wird vorzugsweise in Abwesenheit von Katalysatoren durchgeführt. Es ist jedoch auch möglich, die Halogenierungsreaktion unter Verwendung der Komponente c) katalytisch zu beschleunigen, wobei als Katalysatoren an sich bekannte Brönsted-Säuren oder Lewis-Säuren in Betracht kommen. Geeignete Brönsted-Säuren sind beispielsweise Schwefelsäure, Pyroschwefelsäure, Phosphorsäure, Pyrophosphorsäure und die sauren Salze dieser Säuren, ferner Polyphosphorsäure, Fluorsulfonsäure, Chlorsulfonsäure und Bromsulfonsäure. Lewis-Säuren für das erfindungsgemäße Verfahren sind beispielsweise Thionylchlorid, Sulfurylchlorid, Phosphorpentoxid, Phosphortrichlorid, Phosphoroxychlorid, Aluminiumchlorid, Eisendichlorid, Eisentrichlorid, Zinkchlorid, Bortrifluorid, Bortrichlorid oder Zinntetrachlorid. Die genannten Brönsted- und Lewis-Säuren können sowohl einzeln, als auch als Gemisch verschiedener Brönsted-Säuren, als Gemisch verschiedener Lewis-Säuren und als Gemisch von Brönsted- und Lewis-Säuren eingesetzt werden.

Die Brönsted- oder Lewis-Säure, falls überhaupt, wird beispielsweise in einer Menge von 0,01 bis 3 Mol-%, bevorzugt von 0,1 bis 2 Mol-%, bezogen auf das eingesetzte Isocyanat bzw. Carbamidsäurechlorid, eingesetzt.

Beim erfindungsgemäßen Verfahren kommen im allgemeinen pro Mol an Ausgangskomponente a) 0,2 bis 2 Mol der Komponente b) zum Einsatz. Besonders bevorzugt ist ein Molverhältnis a):b) von 1:0,4 bis 1:1,5. Bei der Herstellung definierter Monosulfonsäurechloride wird in der Regel ein Molverhältnis a):b) von 1:1 bis 1:1,2 eingehalten. Wird der Eintritt von 2 Sulfochlorid-Gruppen in ein Molekül der Ausgangskomponente a) angestrebt, so kommt ein Molverhältnis a):b) von 1:1,8 bis 1:2 zur Anwendung. Grundsätzlich muß darauf

geachtet werden, daß das verwendete Sulfonierungsmittel bei der Reaktion mit dem Diisocyanat weitgehend verbraucht bzw. ein evtl. verwendeter Überschuß entfernt wird, so daß nach beendeter Sulfonierungsreaktion kein Überschuß Sulfonierungsmittel mehr vorhanden ist. Andererseits ist es durchaus möglich, eine nur teilweise Sulfochlorierung der Komponente a) zu bewirken, indem weniger als die äquivalente Menge des genannten Sulfonierungsmittels eingesetzt wird. So kann beispielsweise ein Molverhältnis von 1:0,2 bis 1:0,9 angewendet werden.

Die Komponente c) wird mindestens in Äquivalenz gegenüber der Komponente b) eingesetzt. Im allgemeinen wird die 1,5- bis 10-fach molare Menge, bezogen auf Komponente b), angewandt. Dementsprechend beträgt das Molverhältnis a): c) 1:0,2 bis 1:20, vorzugsweise 1:0,3 bis 1:20. Im Falle der besonders bevorzugten Verwendung von äquimolaren Mengen der Komponenten a) und b) gelangt die Komponente c) vorzugsweise in 1,5- bis 8-fach molaren Mengen, bezogen auf Komponente a) zum Einsatz. Die Verwendung von mehr als 10-fach molaren Mengen der Komponente c), bezogen auf Komponente b), ist für das erfindungsgemäße Verfahren unkritisch, jedoch aus wirtschaftlichen Gründen weniger günstig. Die im Überschuß vorliegende Komponente c) dient als Lösungsmittel für das erfindungsgemäße Verfahren.

Bei der erfindungsgemäßen Reaktion bildet sich aus den Trihalomethyl-Verbindungen die entsprechenden Säurehalogenide, so daß diese Säurehalogenide (beispielsweise Benzoesäurechlorid aus Benzotrichlorid) als Nebenprodukte des erfindungsgemäßen Verfahrens angesehen werden können. Andererseits eignen sich insbesondere diese, der Komponente c) entsprechenden Säurechloride ebenfalls sehr gut als Lösungsmittel für die erfindungsgemäße Halogenierungsreaktion. Weitere erfindungsgemäß in Betracht kommende Lösungsmittel sind beispielsweise Dichlorethan, Titrachlorethan, Nitromethan, Nitrobenzol, Chlorbenzol, o-Dichlorbenzol.

Die erfindungsgemäße, gleichzeitige oder aufeinanderfolgende Umsetzung der Komponente a) mit den Komponenten b) und c) erfolgt im allgemeinen im Temperaturbereich zwischn 0 und 170°C. Die erfindungsgemäße Umsetzung mit der Komponente c), die gleichzeitig mit oder nach der Sulfonierungsreaktion durchgeführt werden kann, erfolgt vorzugsweise im Temperaturbereich zwischen 10 und 150°C, besonders bevorzugt zwischen 20 und 140°C. Im Falle der erfindungsgemäß bevorzugten zweistufigen Reaktionsführung erfolgt im ersten Reaktionsschritt die Sulfonierungsreaktion vorzugsweise im Temperaturbereich zwischen 0 und 80°C und insbesondere zwischen 0 und 40°C, worauf sich die Halogenierungsreaktion innerhalb der genannten Temperaturbereiche anschließt.

Für die Durchführung des erfindungsgemäßen Verfahrens kommen mehere Möglichkeiten in Betracht:

1. Der Komponente a) wird zunächst das Sulfonierungsmittel b) und nach erfolgter Sulfonierungsreaktion die Komponente c) zugesetzt. Bei dieser Verfahrensweise entstehen als Zwischenstufe die Uretdione der entsprechenden Isocyanatoarylsulfonsäuren, die feste unlösliche Verbindungen darstellen. Um die Rührbarkeit zu gewährleisten muß entweder in Gegenwart eines der genannten, insbesondere gegenüber Sulfonierungsmitteln inerten Lösungsmittels und/oder in Gegenwart eines Überschusses der Komponente a) sulfoniert werden. Liegt das Molverhältnis a):b) zwischen 1:0,7 und 1:2, so ist die Mitverwendung eines Lösungsmittels unumgänglich. Im Anschluß an die Sulfonierungsreaktion kann das verwendete Lösungsmittel bzw. das überschüssige Ausgangsisocyanat a) ganz oder auch teilweise entfernt werden, z.B. durch Absaugen, Abschleudern oder auch Destillation. Vor der zweiten Reaktionsstufe sollte das Sulfonierungsmittel entweder verbraucht sein oder, bei Verwendung eines Überschusses der Komponente b), aus dem Reaktionsgemisch beispielsweise durch Auswaschen mit einem inerten Lösungsmittel oder aber auch destillativ entfernt werden. In einem solchen Falle kann auch eine über dem o.g. Molverhältnis a):b) von 1:2 liegende Menge des Sulfonierungsmittels eingesetzt werden.

Die Reihenfolge des Zusammengebens der Ausgangskomponenten a) und b) ist bei einem Molverhältnis a):b) zwischen 1:1,5 und 1:2 nicht kritisch. So ist es beispielsweise möglich, das Sulfonierungsmittel der gegebenenfalls in einem inerten Lösungsmittel

gelösten Komponente a) zuzugeben, oder die Komponente a) in eine Lösung des Sulfonierungsmittels b) in einem derartigen Lösungsmittel zuzumischen. Liegt das Molverhältnis a):b) zwischen 1:0,2 und 1:1,49, so sollte das Sulfonierungsmittel zur vorgelegten Komponente a) zugegeben werden.

Die zweite Stufe des erfindungsgemäßen Verfahrens erfolgt bei dieser ersten Ausführungsform nach Beendigung der Sulfonierungsreaktion durch Vermischen des Sulfonierungsproduktes mit der Komponente c). Nach diesem Durchmischen wird das Reaktionsgemisch solange innerhalb der genannten Temperaturbereiche gehalten, bis kein Halogenwasserstoff mehr entweicht.

2. Gemäß einer denkbaren zweiten Ausführungsform des erfindungsgemäßen Verfahrens wird die Komponente a) zunächst mit der Komponente c) vermischt und dann das Sulfonierungsmittel b) zugegeben. Insbesondere in diesem Fall kann die Komponente c) gleichzeitig die Rolle eines Lösungsmittels übernehmen. Das Reaktionsgemisch wird dann bis zur Beendigung der Halogenwasserstoffbildung innerhalb der genannten Temperaturbereiche gehalten.

3. Gemäß einer dritten Ausführungsform des erfindungsgemäßen Verfahrens wird das Sulfonierungsmittel b) und die Komponente c) gleichzeitig auf die Komponente a) einwirken lassen. So kann man beispielsweise die Komponente a) mit einer Lösung von Chlorsulfonsäure in Benzotrichlorid behandeln. Bei dieser Ausführungsform ist es besonders vorteilhaft, die Mischung aus den Komponenten b) und c) bei etwa

0-20°C der Komponente a) zuzugeben, und die Temperatur dann allmählich auf etwa 140°C zu steigern. Auch hier wird die Reaktion solange fortgesetzt, bis aus dem Reaktionsgemisch kein Halogenwasserstoff mehr entweicht.

Bei allen drei Ausführungsformen des erfindungsgemäßen Verfahrens erfolgt im Anschluß eine möglichst restlose Entfernung des Chlorwasserstoffs, beispielsweise durch eine Wärmebehandlung bei 20-170°C, vorzugsweise 80-170°C.

Die erste Ausführungsform einer stufenweisen Umsetzung ist gegenüber der einstufigen Umsetzung gemäß Ausführungsformen 2 oder 3 bevorzugt.

Nach erfolgter erfindungsgemäßer Umsetzung werden die evtl. verwendete Lösungsmittel, gebildetes Carbon- säurechlorid, sowie überschüssige Komponente c), vorzugsweise durch Destillation entfernt. Der flüssige Destillationsrückstand enthält das Verfahrensprodukt und ist für viele Verwendungszwecke bereits ausreichend rein.

Bei Vorliegen definierter Sulfochloride als Verfahrensprodukt tritt beim Abkühlen des Destillationsrückstandes häufig Kristallisation ein. Eine Reindarstellung der Verfahrensprodukte ist dann durch Umkristallisation möglich.

Es ist ein besonderer Vorteil des erfindungsgemäßen Verfahrens, daß in den meisten Fällen das Rohprodukt ohne Vorreinigung destillativ gereinigt werden kann.

Das erfindungsgemäße Verfahren eignet sich besonders zur Herstellung von 2,4-Diisocyanato-toluol-5-sulfochlorid bzw. Isomerengemischen, welche überwiegend 2,4-Diisocyanato-toluol-5-sulfochlorid enthalten sowie zur Herstellung von 2,4-Diisocyanatotoluol-3,5-disulfochlorid bzw. von Isomeren- oder Homologengemischen, welche neben dem genannten Disulfochlorid 2,4-Diisocyanato-toluol-5-sulfochlorid, 2,4-Diisocyanatotoluol-5-sulfochlorid-3-sulfonsäure enthalten, sowie zur Herstellung von Sulfochlorierungsprodukten von Polyisocyanaten bzw. Polyisocyanatgemischen der Diphenylmethanreihe. Besonders gut geeignet ist das erfindungsgemäße Verfahren auch zur Herstellung von Monosulfochloriden von Diisocyanatotoluol, wenn man nacheinander oder gleichzeitig 2,4-Diisocyanatotoluol bzw. dessen Gemische mit bis zu 35 Gew.-%, bezogen auf Gesamtgemisch, an 2,6-Diisocyanatotoluol nacheinander oder gleichzeitig auf die beschriebene Weise mit Schwefeltrioxid oder Chlorsulfonsäure (Molverhältnis a):b) = 1:1 bis 1:1,2) und mit Benzotrichlorid (Molverhältnis a):c) 1:1,5 bis 1:8) behandelt und den dabei sich bildenden Chlorwasserstoff wie beschrieben aus dem Reaktionsgemisch entfernt.

Das erfindungsgemäße Verfahren bietet gegenüber der Herstellung der Sulfonsäurechloride nach dem Verfahren mit Thionylchlorid oder Phosgen unter Dialkylformamid-Katalyse den Vorteil, daß keine schwer trennbaren Reaktionsgemische auftreten und daß keine potentiell carcinogenen Dialkylcarbamidsäurechloride gebildet werden können.

Ein weiterer Vorteil besteht darin, daß die zu verwendende aromatische Trihalogenmethyl-Verbindung entsprechend ihrer Verfügbarkeit, der Trennbarkeit des Produktgemisches und dem Bedarf an dem simultan entstehenden aromatischen Carbonsäurehalogenid weitgehend variiert werden kann.

Die Durchführbarkeit des erfindungsgemäßen Verfahrens bei relativ niedrigen Temperaturen in guter Ausbeute ist überraschend, da die Sulfonierung von Isocyanaten im allgemeinen zu recht thermostabilen Dimeren führt und unter Annahme dieser Dimeren als Zwischenstufe sowohl der Uretdionring als auch intermediär gebildete Carbamidsäurechlorid wieder gespalten werden muß.

Ein besonderer Vorteil des erfindungsgemäßen Verfahrens besteht darin, daß ausgehend von großtechnisch hergestellten Diisocyanatotoluolen und Diisocyanatodiphenylmethanen auf einfache Weise die entsprechenden Diisocyanatsulfochloride zugänglich sind. Diese, nach dem erfindungsgemäßen Verfahren leicht zugänglichen technischen "TDI-Sulfochloride" bzw. "MDI-Sulfochloride" stellen wertvolle Ausgangsmaterialien zur Herstellung von Polyisocyanat-Polyadditionsprodukten dar.

Die mindestens eine Isocyanat- und mindestens eine Sulfochloridgruppe aufweisenden Verfahrensprodukte sind hochreaktive Verbindungen, die als Zwischenprodukte für eine Vielzahl von Synthesen geeignet sind.

Beispiel 1

In die Lösung von 66,5 g (0,5 Mol) p-Tolyl-isocyanat in 300 ml 1,2-Dichlorethan werden bei 0 bis 6°C 44 g (0,55 Mol) mit St ckstoff verdünntes gasförmiges Schwefeltrioxid ε ngeleitet. Anschließend wird das Reaktionsprodukt vom Dichlorethan abgesaugt, mit Dichlorethan gewaschen und im Exsiccator getrocknet. Laut IR- und Kernresonanz-Spektrum liegt das Uretdion der p-Tolyl-isocyanat-o-sulfonsäure vor.

20 g des Sulfonierungsproduktes werden in 130 ml Benzotrichlorid unter Stickstoff erwärmt, wobei ab 100°C die Abspaltung von Chlorwasserstoff erfolgt. Bei 110°C wird die Lösung klar. Es wird solange weiter erwärmt bis bei 140°C die Chlorwasserstoffabspaltung aufhört. Nach Abdestillieren des Gemisches aus überschüssigem Benzotrichlorid und bei der Reaktion entstehendem Benzoylchlorid wird der Rückstand bei 118°C bis 123°C bei 0,1 Torr destilliert. Man erhält 16,1 g 1-Isocyanato-2-chlorsulfonyl-4-methyl-benzol.
Fp.: 36 bis 38,5°C.

Beispiel 2

Analog Beispiel 1 werden 38,4 g (0,25 Mol) 4-Chlorphenyl-isocyanat mit 20 g Schwefeltrioxid in 350 ml 1,2-Dichlorethan sulfoniert und 40 g des Sulfonierungsproduktes mit 350 ml Benzotrichlorid in das Sulfochlorid übergeführt. Man erhält 32 g einer bei 123 bis 127°C/0,3 Torr destillierenden Flüssigkeit die beim

Abkühlen kristallin erstarrt.
Fp.: 38 bis 42°C.

Aufgrund des IR- und KR-Spektrums handelt es sich um 1-Isocyanato-2-chlorsulfonyl-4-chlorbenzol.

| | | |
|---|---|---|
| Schwefelgehalt: | ber. | 12,7 % |
| | gef. | 12,8 % |
| Chlorgehalt: | ber. | 28,2 % |
| | gef. | 28,3 % |

Beispiel 3

Analog Beispiel 1 werden 46,8 g (0,25 Mol) 4-Trifluormethylphenylisocyanat mit 20 g Schwefeltrioxid in 350 ml 1,2-Dichlorethan sulfoniert. Das noch feuchte Reaktionsprodukt wird mit 350 ml Benzotrichlorid in das Sulfochlorid übergeführt. Nach zweimaliger Destillation werden 25 g einer bei 93 bis 97°C/0,1 Torr siedenden farblosen Flüssigkeit erhalten. Das Produkt ist gaschromatographisch einheitlich. Aufgrund des IR-, KR- und Massenspektrums handelt es sich um 1-Isocyanato-2-chlorsulfonyl-4-trifluormethylbenzol.

Beispiel 4

Analog Beispiel 1 werden 870 g (5,0 Mol) technisches Toluylendiisocyanat (80 % 2,4-, 20 % 2,6-Isomeres) mit 400 g Schwefeltrioxid in 2,4 l 1,2-Dichlorethan sulfoniert. Das noch feuchte Reaktionsprodukt wird mit 2,5 kg Benzotrichlorid bei 82 bis 130°C in das Sulfochlorid übergeführt. Nach Beendigung der Umsetzung und Abdestillieren von Benzotrichlorid und Benzoylchlorid kann das rohe Sulfochlorid ohne weitere Reinigung direkt destilliert werden. Man erhält 1000 g eines Gemisches aus 2,4-Diisocyanatotoluol-5-sulfochlorid und 2,6-Diisocyanatotoluol-5-sulfochlorid.

Beispiel 5

Analog Beispiel 1 werden 750 g (3,0 Mol) 4,4'-Diisocyanatodiphenylmethan mit 52 g Schwefeltrioxid in 750 g 1,2-Dichlorethan teilsulfoniert. Das ausgefallene Reaktionsprodukt wird abgesaugt und 30 g davon noch feucht mit 300 ml Benzotrichlorid bei 100 bis 150°C in das Sulfochlorid übergeführt. Dabei geht der größte Teil des Produktes in Lösung. Zur Aufarbeitung wird vom unlöslichen Rückstand abfiltriert und überschüssiges Benzotrichlorid sowie Benzoylchlorid vollständig abdestilliert.

Man erhält ein festes nicht destillierbares rohes Sulfochlorid des 4,4'-Diisocyanatodiphenylmethans.

| | | |
|---|---|---|
| Schwefelgehalt: | ber. | 9,2 % |
| | gef. | 9,0 % |
| Chlorgehalt: | ber. | 10,2 % |
| | gef. | 8,9 % |

Beispiel 6

Es wird gemäß Beispiel 1 verfahren, jedoch wird vor der Sulfonierung das eingesetzte p-Tolylisocyanat durch Einleiten von Chlorwasserstoff bei 2 bis 3°C in das Carbamidsäurechlorid umgewandelt. Anschließend wird die gebildete Suspension sulfoniert. Das Ergebnis entspricht dem des Beispiels 1.

Beispiel 7

In eine Lösung von 125 g (0,5 Mol) 4,4'-Diisocyanatodiphenylmethan in 700 ml 1,2-Dichlorethan werden nacheinander 40 g (1,1 Mol) Chlorwasserstoff (Bildung des Bis-carbamidsäurechlorids) und 40 g (0,5 Mol) Schwefeltrioxid bei 0 bis 10°C eingeleitet. Anschließend wird die Suspension langsam bis zum Siedepunkt des Dichlorethans erwärmt, wobei ab ca. 50°C starke Chlorwasserstoff-Abspaltung erfolgt. Das Dichlorethan wird weitgehend abdestilliert und dem Rückstand 350 ml Benzotrichlorid zugesetzt. Unter weiterer Chlorwasserstoff-Abspaltung wird die Temperatur langsam auf 130°C gesteigert, bis keine Gasentwicklung mehr erfolgt.

Von unlöslichem Rückstand wird heiß abfiltriert und überschüssiges Benzotrichlorid sowie Benzoylchlorid im Vakuum abdestilliert. Der überwiegend kristalline Rückstand ist laut Massenspektrum und Dünnschichtchromatographie des Umsetzungsproduktes mit Dimethylamin ein Gemisch aus wenig unverändertem 4,4'-Diisocyanatodiphenylmethan sowie dessen Mono- und Disulfochlorid.

Patentansprüche

1) Verfahren zur Herstellung von aromatisch gebundene Sulfochloridgruppen aufweisenden aromatischen Isocyanaten, dadurch gekennzeichnet, daß man

Isocyanate oder Carbamidsäurechloride a), die mindestens eine aromatisch gebundene Isocyanatgruppe und/oder mindestens eine aromatisch gebundene Carbamidsäurechloridgruppe aufweisen

nacheinander oder gleichzeitig bei 0-170°C mit

einem Sulfonierungsmittel b) und

organischen Verbindungen c) mit mindestens einer aromatisch gebundenen Trihalogenmethyl-Gruppe

behandelt und bei 20-170°C Chlorwasserstoff aus dem Reaktionsgemisch entfernt.

2) Verfahren gemäß Anspruch 1, dadurch gekennzeichnet, daß man als Reaktionspartner a) Isocyanate ausgewählt aus der Gruppe bestehend aus Phenylisocyanat, p-Tolylisocyanat, m-Tolylisocyanat, p-Chlorphenylisocyanat, m-Chlorphenylisocyanat, p-Bromphenylisocyanat, p-Methoxyphenylisocyanat, p-Ethoxyphenylisocyanat, p-Trifluormethylphenylisocyanat, m-Trifluormethylphenylisocyanat, 2,4-Diisocyanatotoluol, dessen technischen Gemische mit 2,6-Diisocyanatotoluol, 4,4'-Diisocyanatodiphenylmethan und dessen technischen Gemische mit 2,2'-Diisocyanatodiphenylmethan, 2,4'-Diisocyanatodiphenylmethan und/oder höher als difunktionellen Polyisocyanaten der Diphenylmethanreihe oder die diesen Isocyanaten entsprechenden Carbamidsäurechloride verwendet.

3) Verfahren gemäß Ansprüchen 1 und 2, dadurch gekennzeichnet, daß man als Reaktionspartner b) Schwefeltrioxid oder Chlorsulfonsäure verwendet.

4) Verfahren gemäß Ansprüchen 1 bis 3, dadurch gekennzeichnet, daß man als Reaktionspartner c) gegebenenfalls inerte Substituenten aufweisendes Benzotrichlorid oder Benzotribromid verwendet.

5) Verfahren gemäß Ansprüchen 1 bis 4, dadurch gekennzeichnet, daß man für jedes Mol der Komponente a) 0,2-2,0 Mol der Komponente b) und 0,2-20 Mol der Komponente c) einsetzt.